# EUROPEAN PATENT SPECIFICATION

(11) **EP 0 681 464 B1**
(45) Date of publication and mention of the grant of the patent: **26.01.2000**
(21) Application number: 95900761.8
(22) Date of filing: 18.11.1994
(51) Int. Cl.: A61K 7/13

(54) **CATIONIC DYES FOR KERATIN-CONTAINING FIBRES**
KATIONISCHE FARBSTOFFEN FÜR KERATINHALTIGEN FASER
COLORANTS CATIONIQUES POUR FIBRES KERATINIQUES

(30) Priority: 30.11.1993 CH 356893
(43) Date of publication of application: 15.11.1995
(73) Proprietor: Ciba Specialty Chemicals Holding Inc., 4057 Basel (CH)
(72) Inventor: MÖCKLI, Peter, D-4124 Schönenbuch (CH)
(86) International application number: EP9403826
(87) International publication number: WO9515144

(56) References cited:
- DE-A- 4 137 005
- FR-A- 2 140 205
- FR-A- 2 282 860

## Description

The present invention relates to a process for dyeing keratin-containing fibres, in particular human hair, with cationic dyes.

By far the largest proportion of all hair dyeings are carried out, even today, using so-called "oxidation colours", which involves applying small, colourless precursor molecules to the hair and reacting them by an oxidation process to form larger, coloured molecules. Although this produces the most durable ("permanent") colourings, increasing reservations are being voiced about possible toxicological risks posed not only by the substances used as starting materials but also by the oxidation intermediate and end products, whose precise composition is virtually uncontrollable. Further disadvantages are the relatively complicated use and in particular also the hair damage due to the aggressive chemicals used.

The other, so-called "semipermanent" and "temporary" colourings involve the use of ready-prepared dyes, specifically primarily uncharged disperse dyes and relatively sparingly water-soluble acid dyes. Cationic dyes, by contrast, play only a very minor part. As the terms "semipermanent" and "temporary" indicate, these colourings only have a medium to poor fastness level. Especially the cationic dyes have a reputation for poor hydrolysis and light resistance and for uneven colouring of the hair, for example between root and tip (see: John F. Corbett: The Chemistry of Hair-care Products, JSDC August 1976, p. 290). In addition, the known cationic dyes have an insufficient build-up; i.e., even if increased amounts are used, it is impossible to exceed a certain, relatively low, colour strength. For instance, it is not possible to achieve a deep black coloration with the most important cationic hair dyes Basic Yellow 57, Basic Red 76, Basic Blue 99, Basic Brown 16 and Basic Brown 17 which are used in practice. For the same reason it is difficult to tint relatively dark natural hair with these dyes.

It has now been found that surprisingly cationic dyes of the below-indicated formulae have none of these disadvantages. They can be used to achieve in a very simple way and under gentle conditions very deep dyeings having excellent light, shampooing and crock fastness properties. Owing to their extremely clean shades, they also extend the range of possible mixed shades considerably, especially in the direction of the increasingly important brilliant fashion colours.

The present invention accordingly provides a process for dyeing keratin-containing fibres, which comprises treating the fibres with a dye of the formula 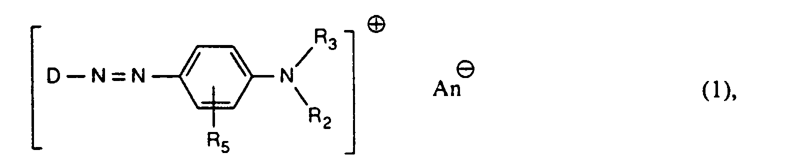 where
D is the radical of a diazo component of the formula 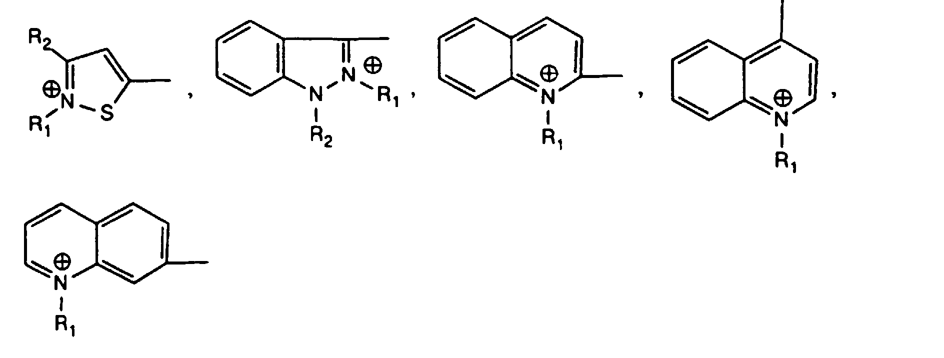
R₁ is unsubstituted or OH-, C₁-C₄alkoxy-, halogen-, CN-, amino-, C₁-C₄monoalkylamino- or di-C₁-C₄alkylamino-substituted C₁-C₄alkyl,
R₂ and R₃ are independently of each other hydrogen or unsubstituted or OH-, C₁-C₄alkoxy-, halogen-, CN-, amino-, C₁-C₄monoalkylamino- or di-C₁-C₄alkylamino-substituted C₁-C₄alkyl, or
R₃ and R₂ are together with the nitrogen atom joining them together a 5- or 6-membered ring,
R₅ is hydrogen, C₁-C₄alkoxy, halogen, C₁-C₄alkyl or C₁-C₄alkylcarbonylamino, or
R₅ and R₂ are together with the nitrogen and carbon atoms joining them together a 5- or 6-membered ring, and
An^{⊖} is a colourless anion.

For the purposes of the present invention, alkyl radicals are generally straight-chain or branched C₁-C₄alkyl groups. Suitable are for example methyl, ethyl, n-propyl, isopropyl, n-butyl, sec-butyl or tert-butyl.

Suitable alkoxy radicals are those having 1 to 4 carbon atoms, e.g. methoxy, ethoxy, propoxy, isopropoxy, n-butoxy, isobutoxy or tert-butoxy.

Halogen is to be understood as meaning fluorine, bromine, iodine or in particular chlorine.

If R₅ and R₂ are combined with the nitrogen atom and the two carbon atoms joining them together into a 5- or 6-membered ring, this ring may contain a further heteroatom, for example oxygen or sulfur. Moreover, the ring may be substituted, for example by hydroxyl, alkoxy, alkyl, halogen, CN or phenyl, or carry a further fused-on benzene ring. Preferred rings formed by R₅, R₂, the linking carbon atoms and the nitrogen atom are pyrroline, dihydrooxazine and di- or tetrahydropyridine rings carrying 0 to 4 methyl groups.

R₂ and R₃ can also combine with the nitrogen atom joining them together to form a piperidine, morpholine or piperazine radical. The piperazine radical can be substituted at the nitrogen atom which is not bonded to the phenyl ring by C₁-C₄alkyl or hydroxy-C₁-C₄alkyl or amino-C₁-C₄alkyl. The preferred substituent is hydroxyethyl.

Suitable anions An^{⊖} include organic as well as inorganic anions, for example chloride, bromide, sulfate, hydrogensulfate, methosulfate, phosphate, borotetrafluoride, carbonate, bicarbonate, oxalate, formate, acetate, propionate, lactate or complex anions, such as the anion of zinc chloride double salts.

The anion is generally given by the method of preparation. Preferred anions are chloride, sulfate, hydrogensulfate, methosulfate, phosphate, formate, acetate or lactate.

Of the dyes of the formula (1), particular preference is given to those where R₁ is unsubstituted C₁-C₄alkyl, especially methyl or ethyl.

Particular preference is also given to dyes of the formula (1) where R₂ and R₃ are independently of each other hydrogen or unsubstituted C₁-C₄alkyl, especially methyl or ethyl, and to those where R₅ is hydrogen, methoxy, ethoxy, chlorine, methyl or ethyl.

The dyes of the formula (1) are known or can be prepared in a manner known per se.

The present invention further provides a process for dyeing keratin-containing fibres, which comprises treating the fibres with a mixture of at least two cationic dyes of the formula (1).

The processes of the invention are suitable for dyeing furs and also animal and human hair, especially live human hair and domestic animals' hair. As a consequence of the high affinity and the good water solubility of the dyes used, it is possible to do the dyeing at room temperature from aqueous solutions without any assistants whatsoever.

However, it is also possible to use any customary cationic dye assistants used in the dyeing of hair, for example wetting agents, swelling agents, penetration aids or scents. In addition, the dyes can be incorporated into shampoos, creams, gels or pastes. Such cosmetic formulations for dyeing hair comprising at least one dye of the above-indicated formula (1) and also assistants form a further part of the subject-matter of the present invention.

A particular advantage of the dyes used according to the invention for dyeing hair is that, owing to the good build-up of the dyes, the colourings can be prepared by the trichromatic principle; that is, it is possible by using a yellow, a red and a blue dye in suitable mixtures of these dyes to achieve virtually all shades. In addition, exact prediction of the shades obtained is possible, which is not the case with the so-called "oxidation dyes" owing to the varying composition of the end products.

Using colorimetric methods of measurement it is also possible to obtain on natural, unbleached hair predicted shades having regard to the hair's natural colour by determining its yellow, red and blue content and deducting it from the recipe of the desired shade. This is not feasible with the hair dyes previously used.

The colourings obtained are crock-, water-, wash- and light-fast and stable to permanent-deformation agents, for example thioglycolic acid.

In FR-A-2282860 and FR-A-2140205 hair dyeing using specific azo dyes is already shown. The dyes actually disclosed in these references are clearly distinguished from the instantly used dyes.

The Examples which follow illustrate the invention. Parts and percentages are by weight. The temperatures are given in degrees Celsius.

Example 1: A braid-sewn strand of blond, natural, untreated human hair is dyed at 25°C for 5 minutes in a conventional manner with a dye emulsion containing 0.1 % of the blue dye of the formula 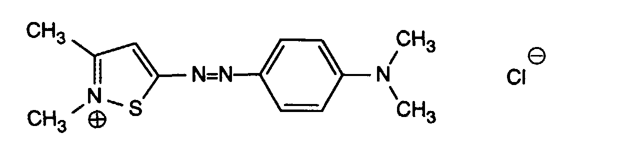
3.5 % of Cetearyl Alcohol
1.0 % of Ceteareth 80
0.5 % of glyceryl mono-di-stearate
3.0 % of stearamide DEA
1.0 % of stearamphopropylsulfonate
0.5 % of polyquaternium-6 and
water to 100 %.

Then the hair is thoroughly rinsed with water and air-dried. The result is an intensive brilliant blue colouring. The light, shampooing and friction fastness properties of the colouring according to the invention are excellent.

Example 2: A 1 % solution of the dye of the formula 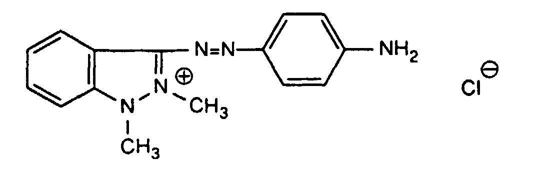 in a surfactant base containing 10 % of cocoamphoglycinate and 90 % of water is applied to Chinese, bleached yak hair at 25°C for 5 minutes, and then the hair is thoroughly rinsed and air-dried. An intensively red colouring is obtained with good light fastness.

Examples 3-7: The method of Examples 1-2 is applied with the dyes listed below in the table, affording colourings on the hair in the specified hues.

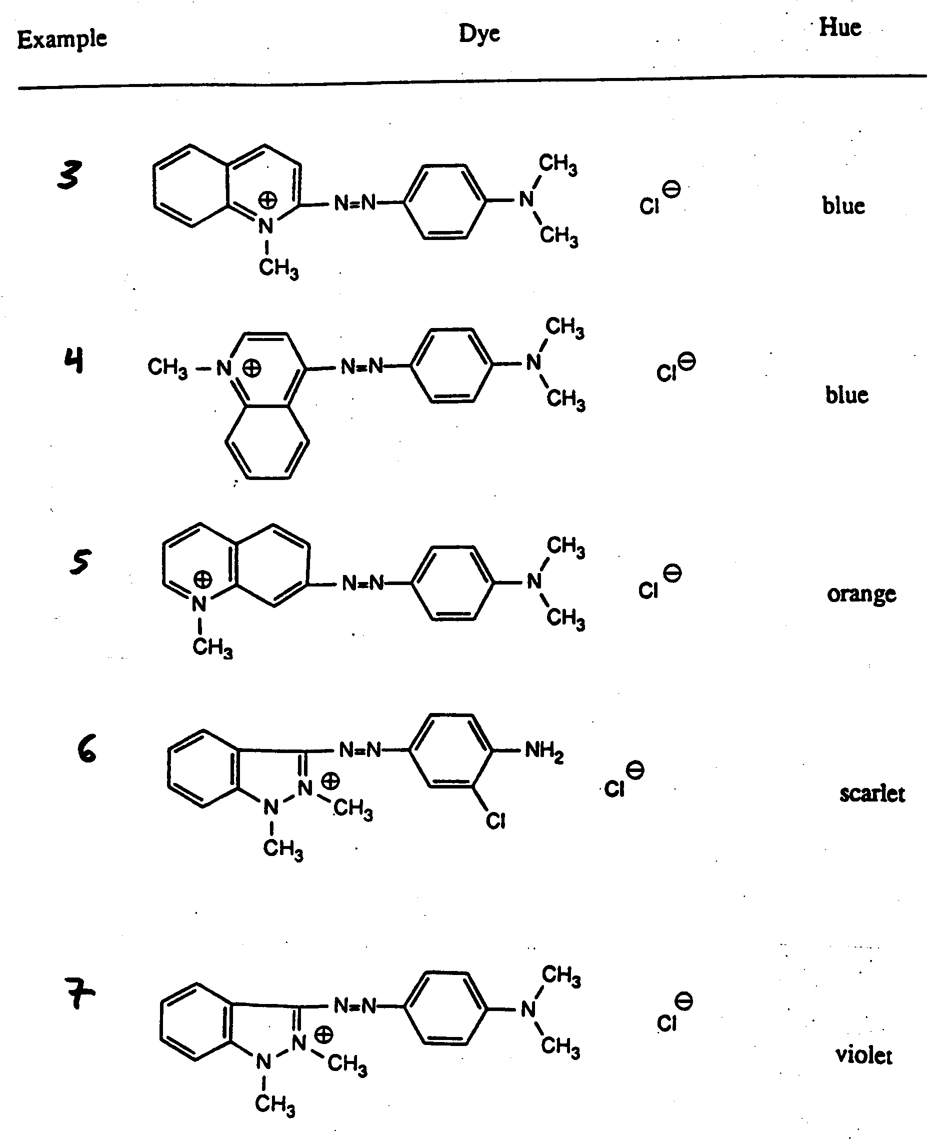

## Claims

1. A process for dyeing keratin-containing fibres, which comprises treating the fibres with a dye of the formula 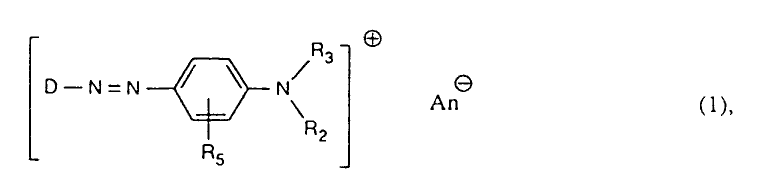 where
D is the radical of a diazo component of the formula 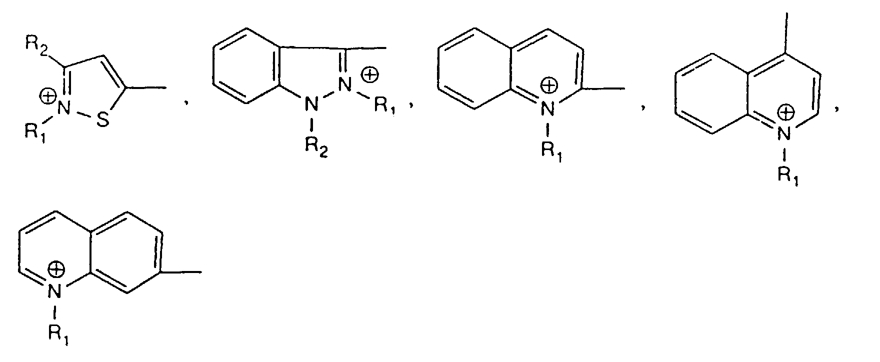
R₁ is unsubstituted or OH-, C₁-C₄alkoxy-, halogen-, CN-, amino-, C₁-C₄monoalkylamino- or di-C₁-C₄alkylamino-substituted C₁-C₄alkyl,
R₂ and R₃ are independently of each other hydrogen or unsubstituted or OH-, C₁-C₄alkoxy-, halogen-, CN-, amino-, C₁-C₄monoalkylamino- or di-C₁-C₄alkylamino-substituted C₁-C₄alkyl, or
R₃ and R₂ are together with the nitrogen atom joining them together a 5- or 6-membered ring,
R₅ is hydrogen, C₁-C₄alkoxy, halogen, C₁-C₄alkyl or C₁-C₄alkylcarbonylamino, or
R₅ and R₂ are together with the nitrogen and carbon atoms joining them together a 5- or 6-membered ring, and
An^{⊖} is a colourless anion.

2. A process according to claim 1, wherein the dye used has the formula (1) where R₁ is unsubstituted C₁-C₄alkyl, especially methyl or ethyl.

3. A process according to any one of claims 1 to 2, wherein the dye used has the formula (1) where R₅ is hydrogen, methoxy, ethoxy, chlorine, methyl or ethyl.

4. A process according to any one of claims 1 to 3 for dyeing live human hair.

5. A process according to any one of claims 1 to 3 for dyeing hairs of domestic animals.

6. A process for dyeing hairs of live animals and humans, which comprises using one of the processes of claims 1 to 3 together with colorimetric methods of measurement to obtain predeterminable shades.

7. A cosmetic formulation for hair dyeing comprising at least one of the dyes of the formulae (1) as set forth in claim 1 and also further assistants.

## Patentansprüche

1. Verfahren zum Färben von keratinhaltigen Fasern, umfassend das Behandeln der Fasern mit einem Farbstoff der Formel worin 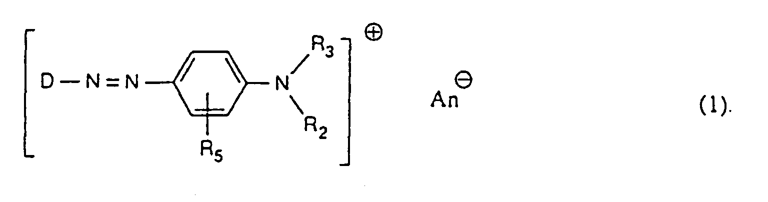
D den 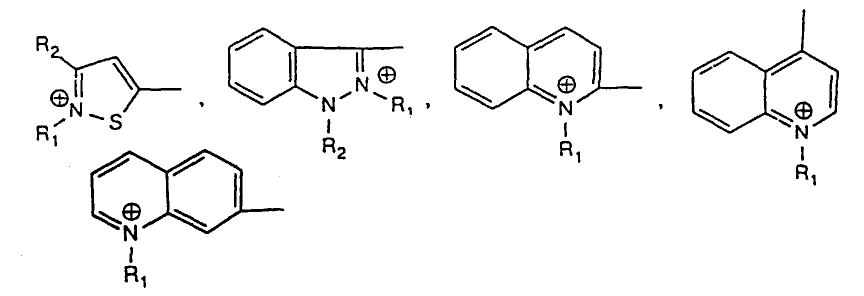 Rest einer Diazokomponente der Formel
R₁ unsubstituiertes oder durch OH, C₁-C₄-Alkoxy, Halogen, CN, Amino, C₁-C₄-Monoalkylamino oder Di-C₁-C₄-Alkylamino substituiertes C₁-C₄-Alkyl,
R₂ und R₃ unabhängig voneinander je Wasserstoff, unsubstituiertes oder durch
OH, C₁-C₄-Alkoxy, Halogen, CN, Amino, C₁-C₄-Monoalkylamino oder Di-C₁-C₄-Alkylamino substituiertes C₁-C₄-Alkyl, oder
R₂ und R₃ zusammen mit dem sie verbindenden N-Atom einen 5- oder 6- gliedrigen Ring,
R₅ Wasserstoff, C₁-C₄-Alkoxy, Halogen, C₁-C₄-Alkyl oder C₁-C₄-Alkylcarbonylamino, oder
R₅ und R₂ zusammen mit den sie verbindenden N- und C-Atomen einen 5- oder 6-gliedrigen Ring, und
An^{⊖} ein farbloses Anion bedeutet.

2. Verfahren gemäss Anspruch 1, wobei man einen Farbstoff der Formel (1) verwendet, worin R₁ unsubstituiertes C₁-C₄-Alkyl, vor allem Methyl oder Ethyl, bedeutet.

3. Verfahren gemäss einem der Ansprüche 1 bis 2, wobei man einen Farbstoff der Formel (1) verwendet, worin R₅ Wasserstoff, Methoxy, Ethoxy, Chlor, Methyl oder Ethyl bedeutet.

4. Verfahren gemäss einem der Ansprüche 1 bis 3 zum Färben von lebendem menschlichem Haar.

5. Verfahren gemäss einem der Ansprüche 1 bis 3 zum Färben von Haaren von Haustieren.

6. Verfahren zum Färben von Haaren an lebenden Tieren und Menschen, wobei eines der Verfahren gemäss den Ansprüchen 1 bis 3 unter Einsatz colorimetrischer Messverfahren zur Erzielung vorausbestimmbarer Farbnuancen angewendet wird.

7. Kosmetische Formulierung zur Haarfärbung, enthaltend mindestens einen der Farbstoffe der Formel (1) gemäss Anspruch 1 sowie weitere Hilfsmittel.

## Revendications

1. Procédé pour teindre des fibres contenant de la kératine, qui consiste à traiter les fibres avec un colorant de formule 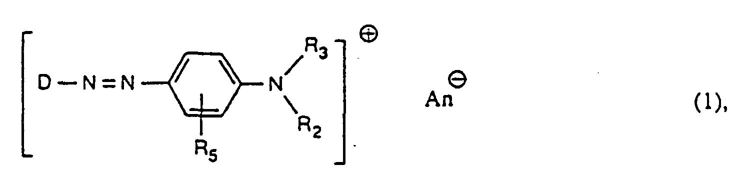 où
D est le radical d'un composant diazoïque, ayant la formule 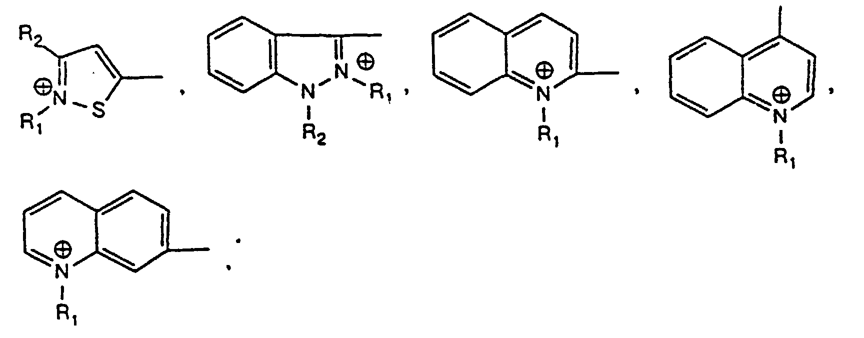
R₁ est un groupe alkyle en C₁-C₄ non substitué ou substitué par un ou plusieurs groupes OH, alcoxy en C₁-C₄, halogéno, CN, amino, mono(alkyle en C₁-C₄)amino ou di(alkyle en C₁-C₄)amino,
R₂ et R₃ sont chacun, indépendamment de l'autre, l'hydrogène ou un groupe alkyle en C₁-C₄ non substitué ou substitué par un ou plusieurs groupes OH, alcoxy en C₁-C₄, halogéno, CN, amino, mono(alkyle en C₁-C₄)amino ou di(alkyle en C₁-C₄)amino, ou bien
R₃ et R₂ forment ensemble, avec l'atome d'azote qui les relie, un cycle penta- ou hexagonal,
R₅ est l'hydrogène ou un groupe alcoxy en C₁-C₄, halogéno, alkyle en C₁-C₄ ou (alkyle en C₁-C₄)carbonylamino, ou bien
R₅ et R₂ forment ensemble, avec l'atome d'azote et les atomes de carbone qui les relient, un cycle penta- ou hexagonal, et
An^{⊖} est un anion incolore.

2. Procédé selon la revendication 1, dans lequel le colorant utilisé répond à la formule (1) où R₁ est un groupe alkyle en C₁-C₄ non substitué, notamment méthyle ou éthyle.

3. Procédé selon l'une quelconque des revendications 1 et 2, dans lequel le colorant utilisé répond à la formule (1) où R₅ est l'hydrogène ou un groupe méthoxy, éthoxy, chloro, méthyle ou éthyle.

4. Procédé selon l'une quelconque des revendications 1 à 3, pour teindre des cheveux vivants.

5. Procédé selon l'une quelconque des revendications 1 à 3, pour teindre des poils d'animaux domestiques.

6. Procédé pour teindre des cheveux et des poils d'animaux vivants, qui consiste à utiliser l'un des procédés des revendications 1 à 3 en association avec des techniques de mesure colorimétriques afin d'obtenir des nuances prédéterminables.

7. Formulation cosmétique pour teinture capillaire, comprenant au moins l'un des colorants de formule (1) tels que définis dans la revendication 1, ainsi que des agents auxiliaires supplémentaires.
